# EUROPEAN PATENT SPECIFICATION

(11) **EP 4 152 935 B1**
(45) Date of publication and mention of the grant of the patent: **17.06.2026**
(21) Application number: 20730394.2
(22) Date of filing: 18.05.2020
(51) Int. Cl.: A01N 59/16, C09D 5/00, A61L 2/238, A01N 59/20, A01P 1/00

(54) **TEMPORARY COVER LAYER, METHOD AND USE**
TEMPORÄRE DECKSCHICHT, VERFAHREN UND VERWENDUNG
COUCHE DE REVÊTEMENT TEMPORAIRE, PROCÉDÉ ET UTILISATION

(43) Date of publication of application: 29.03.2023
(73) Proprietor: Lainisalo Capital Oü, 10127 Tallinn (EE)
(72) Inventor: LAINISALO, Pertti, 10127 Tallinn (EE)
(74) Representative: Papula Oy
(86) International application number: PCT/EP2020/063844
(87) International publication number: WO 2021/233523

(56) References cited:
- EP-A1- 2 098 664
- WO-A1-2005/056699
- WO-A1-2020/231955
- WO-A1-2020/237004
- WO-A1-89/12144
- CN-A- 106 893 520
- CN-A- 107 815 260
- US-A- 5 226 380
- US-A1- 2004 266 546
- US-A1- 2012 171 276
- US-A1- 2012 261 054
- US-A1- 2016 262 380

## Description

### FIELD

The present invention relates to removing and/or inactivating micro-organisms such as viruses and bacteria on surfaces. This includes removing and/or inactivating micro-organisms such as viruses and bacteria on touching surfaces and underwater anti-fouling.

### BACKGROUND

Micro-organisms are prevalent in both built and natural environments and some of them cause adverse effects on people or their equipment. As an example, it is known that micro-organisms can be transmitted from person to person through indirect contact with touching surfaces mediating the transmission. Consequently, efforts to suppress such transmission generally involve cleaning and disinfection of such surfaces. This is a labor-intensive and costly process, which in practice has a limited efficacy. The same applies to cleaning equipment for ensuring their function.

Several documents may be cited here. WO2020/231955 A1 discloses a ductile and flexible antimicrobial adhesive film and WO2020/237004 A1 discloses handle sleeves comprising bioactive material and being able to reversibly couple to a handle. The aforementioned two documents relate to earlier applications and are published after the filing date of the present application. US20120171276 A1 discloses a virus inactivating sheet that can inactivate viruses adhering thereto regardless of whether the viruses have an envelope. US20160262380 A1 discloses a biocidal article. CN106893520 A discloses a thermosensitive pressure sensitive tape for sterilization. US20120261054 A1 discloses a covering for objects for preventing the transmission of infections. CN107815260 A discloses an environment-friendly bactericidal adhesive tape. EP2098664 A1 discloses a hand grip cover with antibacterial effect in base material. US20040266546 A1 discloses an antimicrobial grip for handles on equipment. WO2005/056699 A1 discloses a marine anti-bio-fouling coating. US5226380 A discloses a covering material for underwater objects. WO89/12144 A1 discloses a substantially continuous copper-containing marine antifouling surface.

### OBJECTIVE

An objective is to alleviate the disadvantages mentioned above.

In particular, it is an objective to provide a solution for reducing indirect transmission of micro-organisms, such as viruses and bacteria, through touching surfaces.

Additionally, it is an objective to provide a solution for reducing biofouling of underwater objects.

Moreover, it is an objective to provide a solution for removing and/or inactivating the micro-organisms on surfaces that does not require separate cleaning or disinfection of the surface, for example by utilizing chemicals.

Finally, it is an objective to provide a solution that can be used cost- and labor-efficiently and where the active ingredient is a natural material, thereby allowing the solution to be provided without the risks of synthetic products.

### SUMMARY

In accordance with the present disclosure, it has been found that micro-organisms on a surface may be efficiently removed and/or inactivated, when the surface is covered by a layer comprising copper and/or silver. This may be used to reduce the half-life of the micro-organisms on the surface. Removing may herein include destroying and/or expelling. Inactivating may include modifying virus and/or bacteria to remove their viral or bacterial function. The capability for removal and/or inactivation of micro-organisms may herein be also referred as "hygienic properties". While the micro-organisms in question may be any microscopic organisms, including fungi, particularly advantageous practical utilization has been found when the micro-organisms are viruses and/or bacteria. All types of viruses are included in the micro-organisms in accordance with the present disclosure. In particular, the viruses may be respiratory viruses such as corona viruses, including SARS-CoV-1 and/or SARS-CoV-2.

In accordance with the present disclosure, it has been found that the hygienic properties of copper and/or silver can be efficiently utilized by providing a temporary cover layer for objects (herein also "cover layer"). In particular, the temporary cover layer can be arranged for covering a surface of an object. The surface can be a touching surface, which is by its nature frequently touched by people and therefore provide an efficient channel for transmission of micro-organisms. Consequently, the temporary cover layer decreasing the half-life of micro-organisms on the touching surface may be markedly reduce the transmission of the micro-organisms from person to person. On the other hand, the surface can be exposed to micro-organisms, which necessitate cleaning of the surface, for example to prevent the object such as a piece of equipment from deteriorating.

According to a first aspect, a temporary cover layer for removing and/or inactivating micro-organisms, such as viruses and/or bacteria, comprises a body having an attachment surface for temporarily attaching the temporary cover layer to a surface of an object. This allows the cover layer to be used in various applications for covering the surface of the object, in particular its touching surface. The temporary cover layer can also be replaced after a period of use, which has been found to improve its hygienic properties. The body also has a face, for covering the surface of the object, comprising an active ingredient. The active ingredient comprises a plurality of particles having a core comprising copper and a coating comprising silver. This allows the cover layer to remove and/or inactivate micro-organisms from its surface, thereby reducing the half-life of the micro-organisms on the surface of the object. On the face of the body, the active ingredient may come into direct contact with the micro-organisms, thereby providing the hygienic properties of the cover layer. Utilizing copper and silver, which are natural materials, allows the hygienic properties of the face to be provided utilizing natural materials so that any risks from synthetic materials can be mitigated or removed altogether. In a simple form, the active ingredient may consist only of copper and silver, for example as pure or coated particles or a mixture thereof. Further, the structure of the cover layer allows the cover layer to be provided as a film, which may be made substantially imperceptible for use. The temporary cover layer may be shaped to match the shape of the surface of the object. This is particularly effective for covering the touching surface of the object, since the touching surface may take various shapes, for example in handles. The cover layer may also be shaped to cover the object from two or more sides and/or to envelop the object from opposite sides. The durability of the cover layer may be easily controlled by varying the amount of the active ingredient. As the face comprising the active ingredient is eroded, new active ingredient can be exposed until the exposable active ingredient in the body is completely worn off.

In an embodiment, the attachment surface is an adhesive surface. This allows the cover layer to be easily and quickly fixed in position with respect to the object. The cover layer can thereby be provided as a self-adhesive cover layer, such as a tape. In a further embodiment, the adhesive surface is formed of removable adhesive for detaching the temporary cover layer from the object. This allows the cover layer to be easily and quickly removed or replaced, for example without tools. Removable adhesive may also allow the cover layer to be detached from the object, for example by pulling, without ripping the cover layer. Prior to installation, the temporary cover layer may comprise a backing surface element such as a backing paper attached to the adhesive surface for preventing the adhesive surface from sticking to its surroundings.

In an embodiment, the body comprises a supporting layer and a coating applied on the supporting layer. The supporting layer comprises the attachment surface, whereas the coating comprises the active ingredient and can thereby form the face of the body. This structure allows the cover layer to be formed efficiently as a coated body, such as a coated film, wherein the hygienic properties of the cover layer can be efficiently controlled by controlling the properties of the coating. Also, the properties of the coating may be efficiently controlled, for example by automized coating. The supporting layer may be a piece of tape so that the cover layer may advantageously be fabricated as a coated tape.

In a further embodiment, the coating comprises a binder for binding the active ingredient to the supporting layer. The binder may be provided as a substance that hardens during the formation of the coating for binding the active ingredient to the supporting layer. The hardening may take place through a chemical and/or a physical process. The coating may consist of the binder and the active ingredient, which may be mixed together or layered, at least substantially, with the binder setting between the supporting layer and the active ingredient. In an embodiment, the binder is selected from a group consisting of an alkyd, polymethyl methacrylate (PMMA) and polyurethane. Specific binders have been found to have application-specific advantages. Alkyd may be used to act as a mild binder with cost-benefits, in particular for domestic applications. PMMA may be used for improved durability under abrasion and ultraviolet light, making it particularly advantageous for outdoor use. Polyurethane may be used for improved durability under abrasion, ultraviolet light, chemicals and humidity, making it particularly advantageous not only for outdoor use but also for more demanding applications such as bathing sites and medical sites. Polyurethane may also be used to bind a relatively thick layer of active ingredient thereon.

In an embodiment, the coating has a thickness of 100 micrometers or less, for example 10-100 micrometers. This has been found to provide a balance in being cost-effective and easy to manufacture for a temporary cover layer, while providing sufficient hygienic properties for an extended period of time and allowing the cover layer to be shaped in use for various applications. A thinner coating may be used to improve the flexibility of the cover layer, when necessary.

In an embodiment, the coating has a thickness of 10 micrometers or more, for example 20-25 micrometers or more. This has been found to provide a balance in being cost-effective and easy to manufacture for a temporary cover layer, while providing sufficient hygienic properties for an extended period of time. A thicker coating may be used to improve the durability of the hygienic properties as it allows a thicker layer of active ingredient which can withstand an increased number of touches before wearing off. It may also be used to improve the electric conductivity of the face, which may in some applications, for example for viruses, improve the hygienic properties of the cover layer.

In an embodiment, the face consists of the active ingredient for removing and/or inactivating the micro-organisms. This allows a surface layer to be formed on the body consisting of the active ingredient, which surface layer can be homogeneous or substantially homogeneous, also when it comprises a mixture of different kinds of particles. In particular, the surface layer may be metallic so that its electrical conductivity is high.

Depending on the application, different compositions of the active ingredient may improve the hygienic properties and/or the material characteristics of the cover layer. Different compositions may also be used for different kind of micro-organisms such as viruses and/or bacteria.

In an embodiment, the active ingredient consists of copper and silver.

In an embodiment, the active ingredient comprises gold. While in some embodiments the amount of gold may be large, it has been found that gold also allows easily providing slight modifications to the hygienic properties of silver and/or copper, thereby allowing for example the hygienic properties of the active ingredient to be adjusted for a specific application and/or micro-organism.

In an embodiment, the active ingredient consists of gold together with copper and silver. In a further embodiment, the amount of gold is 1-10 percentage by weight. Additionally or alternatively, the amount of copper and/or silver may be at least 15-20 percentage by weight each.

In an embodiment, the active ingredient comprises at least 15 percent by weight of copper and at least 15 percent by weight of silver. When copper and silver are used together, specific effects may be provided when both are included with at least 15-20 percent by weight, for example 15-50 percent by weight of silver and 50-85 percent by weight of copper or vice versa. These apply both when the active ingredient consists of copper and silver and when it comprises one or more additional materials such as gold.

In an embodiment, the active ingredient comprises 1-10 percent by weight of gold. This allows adjustment of the hygienic properties of the cover layer.

As indicated above, the active ingredient comprises a plurality of particles having a core comprising copper and a coating comprising silver. The silver in this hybrid may be used to slow down the oxidation process of copper and thereby keep the structure open and efficient for an extended period of time. In a further embodiment, the active ingredient comprises a mixture of particles comprising said plurality of particles and silver particles. Both types of active ingredient may provide advantageous hygienic properties, for example against viruses, while being efficiently usable for temporary cover layer, for example when applied as a pigment with automatized coating such as spray coating. In an embodiment, said plurality of particles comprises 70-90 percent by weight of copper, preferably 75-85 percent, and 10-30 percent by weight of silver, preferably 15-25 percent. When the active ingredient comprises also silver particles, in an embodiment the active ingredient as a whole comprises 50-70 percent by weight of copper, preferably 55-65 percent, and 30-50 percent by weight of silver, preferably 35-45 percent.

In an embodiment, the body is of flexible material. This allows the cover layer to be bent to assume the shape of the surface of the object, such as a handle, when the surface is curved. In particular, the cover layer may be arranged to bend so as to cover the object from two or more sides and/or to envelop the object from opposite sides. While the cover layer may bend in one or two dimensions, particular ease of installation may be provided when the cover layer is formed as a tape for covering a surface that is substantially flat in one dimension.

In an embodiment, the temporary cover layer is for underwater use for anti-fouling. For this purpose, water-durable materials may be used for the body and the attachment surface, for example water-durable binder for the former and/or water-durable adhesive for the latter. The thickness of the body may also be increased to increase the amount of the active ingredient. This allows the durability of the temporary cover layer to be increased for underwater use so that it does not need to be replaced too often. To facilitate prolonged underwater use, the temporary cover layer may be arranged for an expected durability of at least one year, for example 2-3 years, which has been found to provide an effective balance, or more.

According to a second aspect, a patterned sheet comprises one or more temporary cover layers according to the first aspect or any of its embodiments alone or in any combination. This allows each of the one or more temporary cover layers to be patterned as a two-dimensional shape for efficient manufacture and distribution for any shape of surface of an object. Patterned sheets can be transported in a stack in small space. The patterned sheet may be formed as a tape sheet.

In an embodiment, the patterned sheet comprises one or more weakenings for detaching the one or more temporary cover layers. The one or more weakenings allow the one or more cover layers to be easily detached from the patterned sheet for use. The weakenings may be arranged so that the detachment can be performed without tools such as cutting aids, for example by tearing.

According to a third aspect, a roll of tape comprises the temporary cover layer according to the first aspect or any of its embodiments alone or in combination. This allows the temporary cover layer to be easily and quickly installed, for example on railing such as handrails. It also allows the length of the installed cover layer to be easily controlled upon installation. In the roll of tape, the temporary cover layer is provided in a rolled shape so that the body of the cover layer is formed by the elongated body of the tape, i.e. a strip of tape, which may be rolled around a support roll of the roll of tape. The attachment surface of the cover layer may be an adhesive surface, as disclosed above, in which case it may be formed by the adhesive surface of the tape.

According to a fourth aspect, a method of providing a temporary cover layer for removing and/or inactivating micro-organisms, such as viruses and/or bacteria, is disclosed. The method comprises applying a coating comprising an active ingredient to a supporting layer comprising an attachment surface for temporarily attaching the supporting layer to a surface of an object. The temporary cover layer and any of its corresponding parts, such as the coating and the supporting layer, and properties may here be considered to correspond those of the first aspect or any of its embodiments, alone or in any combination. The coating may be applied by an automatized painting device, which allows accurately controlling the thickness of the coating. When the coating comprises a binder and an active ingredient, these may be applied separately. An additional hardening agent may be applied to facilitate the attachment of the coating to the body. The active ingredient comprises a plurality of particles having a core comprising copper and a coating comprising silver.

According to a fifth aspect, an active ingredient comprising a plurality of particles having a core comprising copper and a coating comprising silver is used in a temporary cover layer to remove and/or inactivate micro-organisms, such as viruses and/or bacteria, from a surface of an object. The temporary cover layer may be the temporary cover layer according to the first aspect or any of its embodiments, alone or in any combination.

In an embodiment, the use is anti-fouling use. For this purpose, the temporary cover layer as disclosed herein may be arranged for underwater use.

In general, for underwater use and/or anti-fouling, the temporary cover layer may comprise a binder, as disclosed above, wherein the binder comprises or consists of epoxy. For the same purpose, its attachment surface may be an epoxy-based adhesive for maintaining underwater attachment and removability. For some applications, the temporary cover layer may be adapted to maintain these even after one or more years of use.

It is to be understood that the aspects and embodiments described above may be used in any combination with each other. Several of the aspects and embodiments may be combined together to form a further embodiment of the invention. When same expressions are used in the context of different aspects or embodiments, the corresponding features can be applied in all of the aspects and embodiments.

Some important effects that may be provided by the temporary cover layer are the ease of installation, which may be performed within seconds, and its ability to be shaped for a wide range of touching surfaces. The cover layer may be taken into use relatively effortlessly and quickly and its maintenance can be easily arranged. The temporary cover layer may be efficiently used in private facilities, e.g. at home, or at public facilities. A specific application may also be used to protect equipment, for example by preventing biofouling. While the expected durability of the temporary cover layer may depend, for example, on the application, the environment of use and the number of touches and/or type of wear effected on the cover layer, the amount of the active ingredient may be altered to increase its durability. As an example and depending on application, the temporary cover layer may be arranged for an expected durability of at least 1, 7 or 30 days, so that it can be replaced, when necessary. Naturally, in some applications it may be arranged for even shorter expected durability. For improving hygienic properties of touching surfaces, the temporary cover layer may be arranged for an expected durability of a year or less, for example at most 1-2 months. On the other hand, for example underwater applications such as for anti-fouling the expected durability may be longer as indicated above.

### BRIEF DESCRIPTION OF THE DRAWINGS

The accompanying drawings, which are included to provide a further understanding and constitute a part of this specification, illustrate examples and together with the description help to explain the principles of the disclosure. In the drawings:
**Fig.** 1 illustrates a temporary cover layer according to an example, when installed to an object,
Fig. 2 illustrates a a temporary cover layer according to another example, when installed to an underwater object,
Fig. 3 illustrates a patterned sheet according to an example,
Fig. 4 illustrates a roll of tape according to an example,
Fig. 5a schematically illustrates a temporary cover layer according to an example,
Fig. 5b schematically illustrates a temporary cover layer according to another example, and
Fig. 6 illustrates a method according to an example.

Like references are used to designate equivalent or at least functionally equivalent parts in the accompanying drawings.

### DETAILED DESCRIPTION

The detailed description provided below in connection with the appended drawings is intended as a description of examples and is not intended to represent the only forms in which the example may be constructed or utilized. However, the same or equivalent functions and structures may be accomplished by different examples.

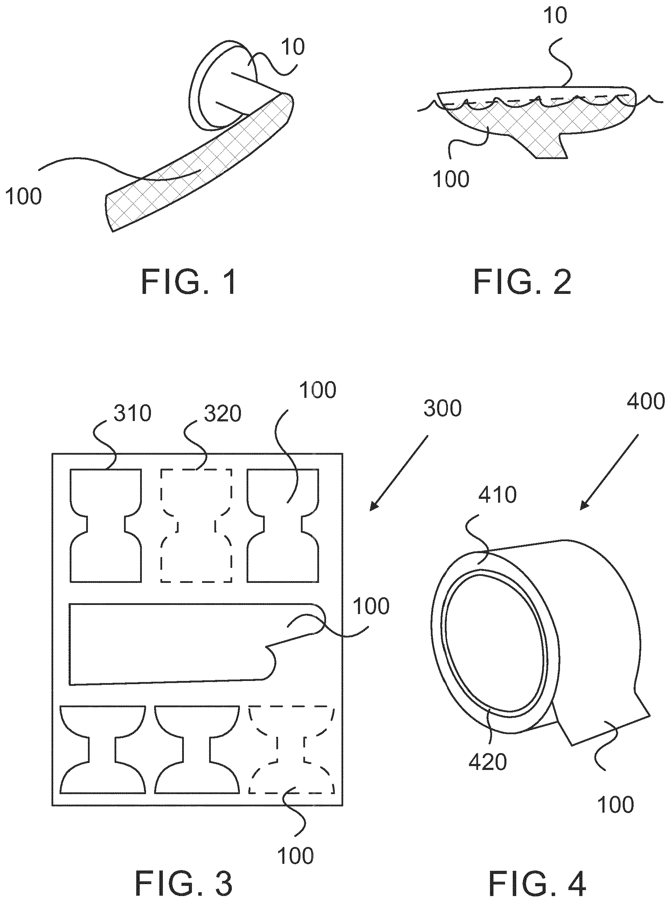

Figure 1 shows an example of a temporary cover layer 100 according to an example, when installed to an object 10. In the illustrated example, the object 10 is a door handle, but in general the object 10 may be any object whose hygienic properties are important. In particular, the object 10 may be or comprise a touching interface such as a handle, a button or a switch, a rail or a pushing surface, for example for opening a door. Correspondingly, the object 10 may comprise a touching surface. The temporary cover layer 100 may be arranged to partially or fully cover the surface of the object 10, in particular the touching surface of the object 10.

The temporary cover layer 100 may be adapted for various uses. A small, non-exhaustive list of potential applications, where the temporary cover layer may be used and for which it may be adapted include household surfaces, door handles including outdoor handles and indoor handles, locks, grip handles, faucets, toilet bowl contact buttons or handles, rails, door bells, holding surfaces of trays, light switches and furniture surfaces and edges. The list further includes elevator buttons, escalator rails, shopping carts and baskets, hand rests, cutleries and tools for taking or transporting food, slide switches, alarm buttons, ATM buttons and surfaces, pens, car handles and buttons and folders. Accordingly, the object 10 may be any of the above.

The cover layer 100 may be adapted for use in public transport including airplanes, subways, buses and taxis. It may adapted for use in employee and/or customer facilities of companies and public sites, for example in hotels. It may further be adapted for demanding environment or use including hospitals, swimming pools, saunas, spas, ice rinks and stadiums, restaurants, retirement and nursing homes, hospices and ambulances.

Figure 2 shows an example of a temporary cover layer 100 according to another example, when installed to an object 10. Here, the object 10, e.g. a boat or a ship, is adapted for underwater use. Correspondingly, the temporary cover layer 100 may be adapted for underwater use. This allows it to be used, for example, for prevention of biofouling. In one embodiment, the temporary cover layer 100 is an anti-fouling cover layer. It may be adapted for attachment to any underwater structure for anti-fouling, including but not restricted to an underwater hull of a vessel such as a boat or a ship, a fishery equipment and a power station.

In accordance with the examples of Figs. 1 and 2, the cover layer 100 may be adapted for preventing person-to-person transmission of micro-organisms and/or protecting equipment, including underwater equipment. As indicated above, the temporary cover layer 100 may be adapted for any application, where hygienic properties of surfaces are important.

Figure 3 shows a patterned sheet 300 according to an example. The patterned sheet 300 comprises one or more temporary cover layers 100, each corresponding to a patterned shape 310 on the patterned sheet. The patterned sheet 300 can be a substantially two-dimensional sheet and it can be substantially formed by material of the one or more temporary cover layers 100. The patterned shapes 310 on one patterned sheet 300 may all be the same, but one patterned sheet 300 may also comprise two or more different patterned shapes 310. Also the same patterned shapes 310 with different sizes may be included in one patterned sheet 300. The patterned sheet 300 can thereby be considered as one larger temporary cover layer 100, from which one or more smaller temporary cover layers 100 may be detached, with the patterned shapes 310 of the one or more smaller temporary cover layer 100 patterned visible on the surface of the patterned sheet 300. The patterning may be performed after the temporary cover layer 100 has been formed, in particular after an active ingredient has been attached into the temporary cover layer 100. For easy production and transportation, the patterned sheet 300 may be substantially rectangular, but other shapes are possible as well. The structure of the temporary cover layer 100 allows the patterned sheet to be provided in any size. In an embodiment, the patterned sheet is of size A5 or A4 or larger. Additionally or alternatively, the patterned sheet can be of size 1m x 2m or smaller allowing it to be efficiently provided with several currently available sheet production techniques. In some examples, the patterned sheet is of size A3, BB or 90cm x 160cm.

The patterned sheet 300 may comprise one or more weakenings 320 such as grooves and/or perforations for detaching the one or more temporary cover layers 100. The weakenings 320 may define one or more lines defining one or more patterned shapes 310. The weakenings 320 may be adapted so that the one or more temporary cover layers may be detached from the patterned sheet 300 without tools, for example by pulling or tearing. The patterned sheet 300 allows conveniently providing temporary cover layers 100 of desired shape readily available for attachment to an object 10.

Figure 4 shows a roll of tape 400 according to an example. The roll of tape 400 comprises a strip of tape 410 rolled upon itself, wherein the tape 410 comprises the temporary cover layer 100. In fact, the temporary cover layer 100 may be formed as a strip of tape 410, in which case the cover layer 100 may itself form the tape 410. The roll of tape 400 may comprise one or more weakenings 320, as described above, for detaching a strip of the temporary cover layer 100 from the tape 410. However, the temporary cover layer 100 may also be substantially homogeneous along the length of the tape, for example for 1-10 meters or more, allowing the temporary cover layer to be separately cut at a desired length. The roll of tape 400 may be effectively applied for covering railings such as handrails, for example those of an escalator. It may be fabricated by a roll-to-roll method. The surface of the temporary cover layer 100 may be fabricated by robotics and/or silk screen printing. The roll of tape 400 may comprise a support roll 420, which may be made of any suitable material such as plastic or paper, including cardboard. The support roll 420 may be substantially circular but other shapes are possible as well. In the roll of tape 400, the tape 410 can be rolled around the support roll 420.

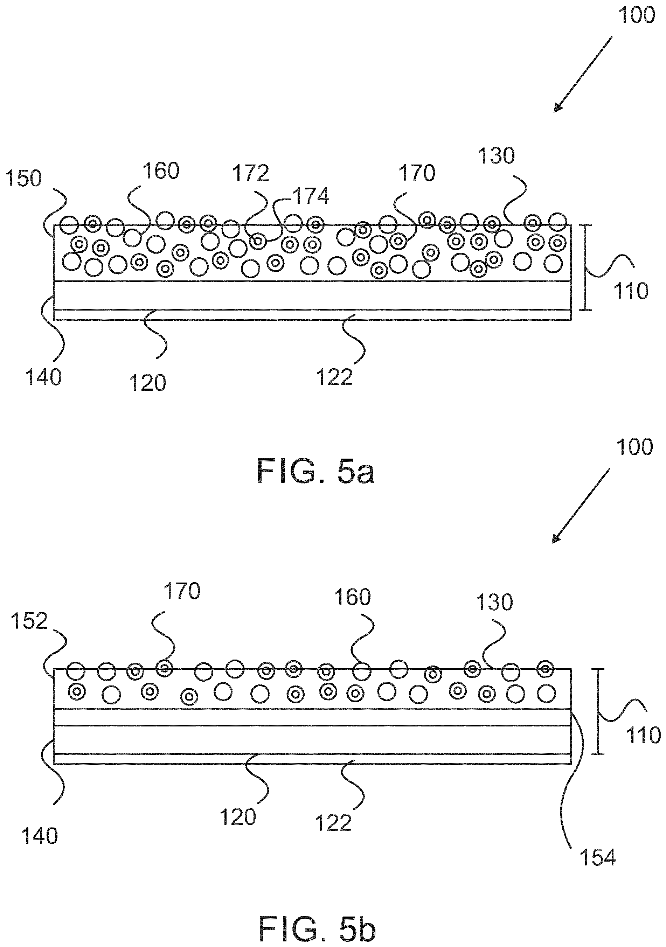

Figure 5a (not in scale) schematically shows a temporary cover layer 100 according to an example. The cover layer 100 comprises and may consist or substantially consist of a body 110 which is adapted for attachment to a surface of an object 10. Herein, "installation" may refer to attachment of the temporary cover layer 100 to the surface of the object 10. The object 10 may be any type of object described above. The body 110 may be adapted for attachment to a touching surface of the object 10 so that it partially or fully covers the touching surface. The body 110 may be thin, for example having a thickness of less than a millimeter. In some embodiments, for example when longer durability is required or when the cover layer 100 is subject to more demanding conditions, the thickness of the body 110 may also be larger. In some embodiments, the body 110 has a thickness of 80-500 micrometers or 100-200 micrometers. The body 110 be of substantially constant thickness but the thickness may also be measured as the maximum thickness of the body 110. The body 110 may be provided as a film. Prior to installation, the body 110 may be substantially flat. It may be adapted to bend to follow the shape of the surface of the object 10. In general, the body 110, like the cover layer 100, may be substantially shaped as a plane, which may be curved or flat.

The body 110 has an attachment surface 120 for temporarily attaching the body 110 and thereby the cover layer 100 to a surface of an object 10. Prior to installation, the attachment surface 120 may be substantially flat. In general, the attachment surface 120, may be substantially shaped as a plane, which may be curved or flat. It may be adapted to bend to follow the shape of the surface of the object 10. It may extend along the whole rear surface of the body 110. The attachment surface 120 may be an adhesive surface, such as a glue surface. The adhesive surface may be formed of removable adhesive, for example of acrylic based adhesive. For removability, the adhesive may have adhesion of less than 1000 N/m. In some embodiments, the adhesion may be 150 N/m or more and/or 500 N/m or less. An adhesive with smaller adhesion, for example 230 N/m may be provided for consumer use, whereas an adhesive with larger adhesion, for example 465-500 N/m may be provided for professional use. The indicated values may refer to values in accordance with FINAT FTM-1 test method with stainless steel. The indicated values may refer to the ultimate adhesion of the adhesive. The adhesive may be provided as a layer of substantially small thickness, such as 10-50 micrometers, for example around 20 micrometers. Prior to installation, the cover layer 100 may comprise a backing surface element 122 such as a backing paper attached to the adhesive surface for preventing the adhesive surface from sticking to its surroundings. The backing surface element may be plastic or paper such as kraft paper.

The body 110 has a face 130, which corresponds to a front surface of the body 110, susceptible to the micro-organisms. The face 130 can be formed on the opposite side of the body 110 with respect to attachment surface 120. The face 130 can be adapted to act as the covered surface of the object 10. It may cover any touching surface and act as a touching surface in its place. Prior to installation, the face 130 may be substantially flat. In general, the face 120, may be substantially shaped as a plane, which may be curved or flat. It may be adapted to bend to follow the shape of the surface of the object 10. It may extend along the whole front surface of the body 110.

The face 130 may comprise or consist of an active ingredient for providing hygienic properties of cover layer 100. As the face 130 forms a front surface of the cover layer 100, this allows the active ingredient to come into direct contact with the micro-organisms. This has been schematically illustrated in figures 5a and 5b by illustrating an uneven face, where particles 160, 170 may partially stick out. In practice, the face 130 may be substantially smooth, even on the microscopic level. In particular, the face 130 may be smooth to the touch. As such, the face 130 can be a substantially smooth plane, for example for providing a touching surface of the body 110. Importantly, on the face 130 the active ingredient may directly interact with the micro-organisms. This may involve chemical and/or physical interactions. The active ingredient may be spread substantially homogeneously in one or both surface dimensions of the body 110, or its face 130.

The active ingredient comprises or consists of copper and silver, for example in particle/particulate form. This allows utilizing the naturally occurring antimicrobial properties such as antibacterial and/or antiviral properties of silver and copper. It may additionally comprise gold, which may also be in particle/particulate form. The active ingredient can be formed solely from a combination of copper and silver, optionally with gold. However, the active ingredient may also include one or more additional materials. The active ingredient may be metallic and correspondingly, the face 130 may be metallic. The active ingredient may be in particle/particulate form or it may comprise metallic particles/particulates. It may also be in any other form, where copper and silver can be provided on the face 130 of the body 110.

As an example, the active ingredient may comprise or consist of a pigment or a combination of pigments, for example used for coating surfaces. A pigment may be a metallic pigment. It may be a coated pigment or an uncoated pigment. The pigment may comprise or consist of any combination of copper, silver and gold. The pigment may be sprayable.

Importantly, the active ingredient may be provided in an electrically conducting form. Consequently, the face 130 may be electrically conductive. In some embodiments, increasing electric conductivity may be used to allow increasing the hygienic properties of the cover layer 100.

In any case, the active ingredient comprises a plurality of particles 170 having a core 172 and a coating 174. The core 172 and/or the coating 174 may be metallic. The core 172 comprises or consist of copper, optionally with one or more of the following: silver and gold. The coating 174 comprises or consists of silver, optionally with one or more of the following: copper and gold. This may be particularly effective for viruses. However, it effectively allows prevention of natural oxidization of copper for the cover layer. The coating may be of the nanometer order in thickness, for example less than 10-100 nanometers or even of the order 0.5-5 nanometers. The coating may comprise or consist of nanoparticles such as silver nanoparticles. The core may comprise or consist of nanoparticles, such as copper nanoparticles. The ratio of copper and silver may vary, but in an embodiment the plurality of particles may comprise 2-10 times the amount of copper with respect to silver by weight, for example substantially four times the amount. Similarly, the plurality of particles may comprise 2-10 times the amount of core with respect to coating by weight, for example substantially four times the amount. The silver-coated copper particles for the active ingredient may be provided using a silver-coated copper conductive coating. However, other silver-coated copper coatings are also possible.

In an embodiment, the active ingredient may comprise metal particles 160, i.e. particles where metal forms the core that is uncoated, or openly exposed. The metal particles may therefore be single-metal or one-component particles. The metal particles may comprise or consist of one or more of the following: copper, silver and gold particles. In an embodiment, comprises or consist of silver particles. The metal particles may be of substantially pure metal. The metal particles for the active ingredient may be provided using a metal conductive coating.

While the active ingredient may be provided in the body 110 in any applicable manner, in an embodiment the body 110 comprises or consists of a supporting layer 140 and a coating 150 applied thereon. The temporary cover layer 100, when in use, may thereby consist of the supporting layer 140 and the coating 150. The coating 150 may be directly applied on the supporting layer 140. It may comprise one or more components, which may be applied separately or as a mixture. The coating 150 may be applied by an automated coating system, for example by automated spray coating. It may be applied, for example, by suction or gravity spray gun. The coating 150 may also be applied by silk screen printing, pad printing, dip coating, rolling paint, robotic painting. The manufacturing method for the cover layer 100 may be roll to roll printing or utilizing spray manipulator.

The supporting layer may be adapted for providing the structural support of the cover layer 100 and for this purpose it may comprise supporting material such as plastic, for example. In an embodiment, the supporting layer 140 is of molded pvc. The supporting layer 140 may be substantially a plane, which may be curved or flat. Upon installation, it may be bent to match the shape of the surface of the object 10. The supporting layer 140 may be formed as a tape such as a label or a sticker. Consequently, the body 110 and the cover layer 100 may be formed as a coated tape. The supporting layer 140, including the attachment surface, may be made relatively thin, for example less than 500 micrometers. In an embodiment, the thickness of the supporting layer is 200 micrometers or less, for example 50-100 micrometers. The supporting layer 140 may be of substantially constant thickness but the thickness may also be measured as the maximum thickness of the supporting layer 140. The thickness of the supporting layer may be determined by ISO 534 method.

The coating 150 may be made thin, having a thickness of less than a millimeter, for example 10-500 micrometers. It has been found that a thickness of 100 micrometers or less, for example 75 micrometers at most, can be used to effectively improve the flexibility of the temporary cover layer 100 for various applications. In some embodiments, the coating 150 has thickness of 25-75 micrometers. The coating 150 may be of substantially constant thickness but the thicknesses indicated may also be measured as the average thickness of the coating 150. With automatized coating, the thickness may have a maximum variation of less than 5-10 micrometers from the average thickness. The coating 150 may be provided as a pure metal coating. The active ingredient may be provided as a pigment of the coating 150.

The temporary cover layer 100 may be a flexible cover layer to adjust to the surface shape of the object 10 or to the shape of the contact surface 100 of the object. For this purpose, the body 100 may be of flexible material, including the supporting layer 140, where present. As an example, the cover layer 100 may be adapted for a bent of at least 90-180 degrees, for example with a radius of curvature smaller than 1-10 millimeters. For some applications, a larger radius of curvature will suffice. As indicated above, a coating 150 of 75-100 micrometers or less may be used to provide efficient flexibility for various applications, in particular for touching surfaces, including handles, switches and buttons.

The coating 150 comprises the active ingredient but it may additionally comprise other materials including a binder, which may be used to facilitate binding of the active ingredient to the supporting layer 140. In an embodiment, the binder comprises or consists of one or more from the following: an alkyd, polymethyl methacrylate (PMMA) and polyurethane. In some applications, epoxy and/or latex may be used. Different binders may be used depending on the desired properties such as mechanical and chemical properties of the coating 150. Some binder may improve, for example, durability, ultraviolet radiation susceptibility and/or flexibility of the cover layer 100. Where thicknesses of coating are indicated above, the indicated flexibility may be obtained with each of the alternatives of binder indicated above. Moreover, for example plastic such as molded-pvc, as indicated above, may be utilized for the supporting layer 140 to provide both the required flexibility and structural strength in any combination with the indicated binders.

The coating 150 may additionally comprise a hardening agent and/or a solvent, or it may be formed utilizing a hardening agent and/or a solvent, which may be removed, for example by evaporation, from the coating 150 upon its formation. Consequently, the indicated thicknesses above may herein be considered also as dry-film thicknesses of a finished temporary cover layer 100, from which intermediate ingredients such as the hardening agent and/or solvent have been removed. The binder and the active ingredient may be applied on the supporting layer 140 separately but also as a mixture. In either case, the coating 150 may be formed as a homogeneous mixture, possibly including the hardening agent and/or the solvent. It may be adapted 150 to form one, two or more layers during its formation so that the coating 150 of the cover layer 100, when finished, consists of one, two or more layers.

Figure 5b (not in scale) schematically shows a temporary cover layer 100 according to another example. In this example, the coating 150 comprises or consists of two layers: a surface layer 152 comprising the active ingredient and a binder layer 154, situated between the surface layer 152 and the supporting layer 140 and binding the surface layer 152 to the supporting layer 140. This may be arranged to result from the formation of the coating 150, even when the binder and the active ingredient are provided as a mixture, even as a homogeneous mixture, during the formation of the coating 150. Any coating technique and material combination known by a person skilled in the art of coating for providing this effect may be utilized. The surface layer 152 comprises the active ingredient. This allows the active ingredient to be effectively provided at the face 130 of the body 110. Even when the active ingredient wears from the face 130, new active ingredient may be thus exposed until the wear-off reaches the binder layer 154. A surface layer 152 may even be formed consisting solely of the active ingredient. The surface layer may extend in one or both dimensions along the whole front surface of the body 110 or, along the whole face 130.

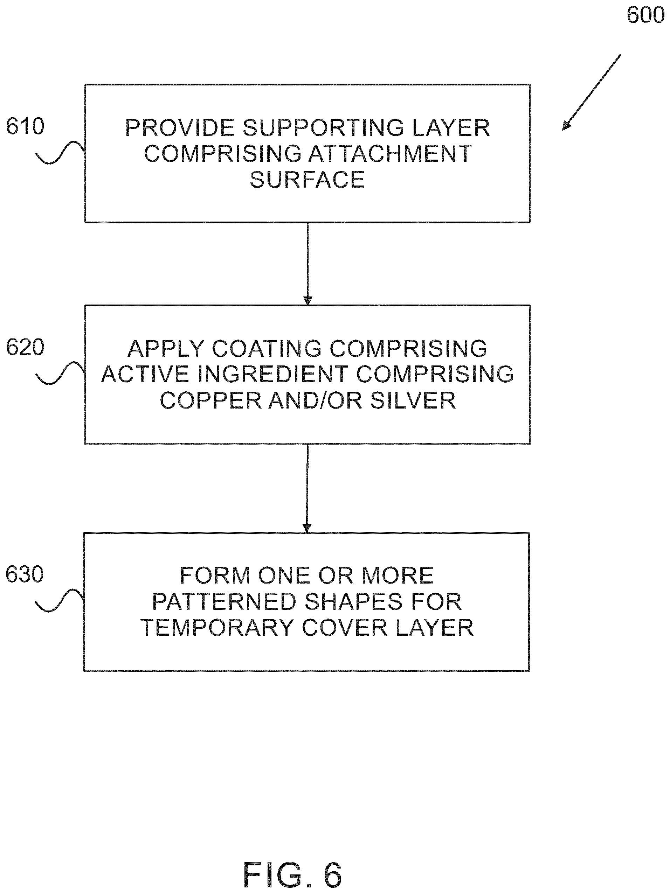

Fig. 6 shows a method 600 according to an example. The method 600 can be used for providing or manufacturing a temporary cover layer 100 for removing and/or inactivating micro-organisms, for example on a surface such as a touching surface of an object 10. The method 600 comprises providing 610 a supporting layer 140 comprising an attachment surface for temporarily attaching the supporting layer to a surface of an object 10. The method then comprises applying 620 a coating 150 comprising an active ingredient to the supporting layer 140. The coating 150 may be applied by any methods, alone or in combination, indicated above. In an embodiment, the active ingredient and/or a binder is applied by spray coating, separately or as a mixture. The coated supporting layer 140 may then be provided as a temporary cover layer 100. Before that, it may naturally also be further processed, where necessary. The active ingredient comprises a plurality of particles having a core comprising copper and a coating comprising silver

The method 600 may further comprise forming 630 one or more patterned shapes 320, as indicated above, for the temporary cover layer 100. This allows the method to be used for providing the patterned sheet 300 comprising one or more temporary cover layers 100. In an embodiment, the one or more patterned shapes 320 are made to the coated supporting layer 140, forming a temporary cover layer 100, i.e. after the coating 150 has been applied to the supporting layer 140. In particular, it can be made after the coating 150 has been hardened to the supporting layer 140, for example after it has been formed as a dry coating. The patterned shapes 320 may be partially or fully cut for easy of the cover layer 100. The method may also be used to provide a roll of tape 400, as described above. It may be fabricated, for example, by a roll-to-roll method.

The term 'comprising' is used herein to mean including the method, blocks or elements identified, but that such blocks or elements do not comprise an exclusive list and a method or apparatus may contain additional blocks or elements.

Although the invention has been described in conjunction with a certain type of apparatus and/or method, it should be understood that the invention is not limited to any certain type of apparatus and/or method. While the present inventions have been described in connection with a number of examples, embodiments and implementations, the present inventions are not so limited, but rather cover various modifications, and equivalent arrangements, which fall within the purview of the claims. Although various examples have been described above with a certain degree of particularity, or with reference to one or more individual embodiments, those skilled in the art could make numerous alterations to the disclosed examples without departing from the scope of this specification.

Notwithstanding what is stated above and in accordance with Article 69 of the European Patent Convention, the extent of the protection conferred by a European patent based on this application shall be determined by the claims. Nevertheless, the description and drawings shall be used to interpret the claims.

## Claims

1. A temporary cover layer for removing and/or inactivating micro-organisms, such as viruses and/or bacteria, comprising a body having an attachment surface for temporarily attaching the temporary cover layer to a surface of an object; wherein the body has a face comprising an active ingredient; **characterized by** the active ingredient comprising a plurality of particles having a core comprising copper and a coating comprising silver.

2. The temporary cover layer according to claim 1, wherein the attachment surface is an adhesive surface, optionally formed of removable adhesive for detaching the temporary cover layer from the object.

3. The temporary cover layer according to any preceding claim, wherein the body comprises a supporting layer and a coating applied on the supporting layer; wherein the supporting layer comprises the attachment surface and the coating comprises the active ingredient.

4. The temporary cover layer according to claim 3, wherein the coating comprises a binder for binding the active ingredient to the supporting layer, the binder being optionally selected from a group consisting of an alkyd, polymethyl methacrylate (PMMA) and polyurethane.

5. The temporary cover layer according to claim 3 or 4, wherein the coating has a thickness of 100 micrometers or less or 10 micrometers or more.

6. The temporary cover layer according to any preceding claim, wherein the face consists of the active ingredient for removing and/or inactivating the micro-organisms.

7. The temporary cover layer according to any preceding claim, wherein the active ingredient consists of copper, silver and, optionally, gold.

8. The temporary cover layer according to any of claims 1-7, wherein the active ingredient comprises gold, preferably 1-10 percent by weight of gold.

9. The temporary cover layer according to any preceding claim, wherein the active ingredient comprises at least 15 percent by weight of copper and at least 15 percent by weight of silver.

10. The temporary cover layer according to any preceding claim, wherein the active ingredient comprises a mixture of particles comprising said plurality of particles and silver particles, said plurality of particles optionally comprising:
- 70-90 percent by weight of copper, preferably 75-85 percent, and
- 10-30 percent by weight of silver, preferably 15-25 percent.

11. The temporary cover layer according to any preceding claim, wherein the body is of flexible material.

12. A patterned sheet comprising one or more temporary cover layers according to any preceding claims and, optionally, one or more weakenings for detaching the one or more temporary cover layers.

13. A roll of tape, wherein the tape comprises the temporary cover layer according to any of claims 1-11.

14. A method of providing a temporary cover layer for removing and/or inactivating micro-organisms, such as viruses and/or bacteria, the method comprising applying a coating comprising an active ingredient comprising plurality of particles having a core comprising copper and a coating comprising silver to a supporting layer comprising an attachment surface for temporarily attaching the supporting layer to a surface of an object.

15. Use of an active ingredient comprising plurality of particles having a core comprising copper and a coating comprising silver in a temporary cover layer to remove and/or inactivate micro-organisms from a surface of an object, wherein the use is optionally anti-fouling use.

## Patentansprüche

1. Temporäre Abdeckschicht zum Entfernen und/oder Inaktivieren von Mikroorganismen, wie Viren und/oder Bakterien, umfassend einen Körper, aufweisend eine Befestigungsoberfläche zum temporären Befestigen der temporären Abdeckschicht an einer Oberfläche eines Objekts, wobei der Körper eine Fläche aufweist, umfassend einen aktiven Inhaltsstoff, **dadurch gekennzeichnet, dass** der aktive Inhaltsstoff eine Vielzahl von Teilchen, aufweisend einen Kern, umfassend Kupfer, und eine Beschichtung, umfassend Silber, aufweist.

2. Temporäre Abdeckschicht nach Anspruch 1, wobei die Befestigungsoberfläche eine klebende Oberfläche ist, die optional aus einem entfernbaren Klebstoff zum Abnehmen der temporären Abdeckschicht von dem Objekt ausgebildet ist.

3. Temporäre Abdeckschicht nach einem der vorhergehenden Ansprüche, wobei der Körper eine Stützschicht und eine Beschichtung, dis auf der Stützschicht aufgebracht ist, umfasst, wobei die Stützschicht die Befestigungsoberfläche umfasst und die Beschichtung den aktiven Inhaltsstoff umfasst.

4. Temporäre Abdeckschicht nach Anspruch 3, wobei die Beschichtung ein Bindemittel zum Binden des aktiven Inhaltsstoffs an die Stützschicht umfasst, wobei das Bindemittel optional aus einer Gruppe ausgewählt ist, die aus einem Alkyd, Polymethylmethacrylat (PMMA) und Polyurethan besteht.

5. Temporäre Abdeckschicht nach Anspruch 3 oder 4, wobei die Beschichtung eine Dicke von 100 Mikrometern oder weniger oder 10 Mikrometern oder mehr aufweist.

6. Temporäre Abdeckschicht nach einem der vorstehenden Ansprüche, wobei die Fläche aus dem aktiven Inhaltsstoff zum Entfernen und/oder Inaktivieren der Mikroorganismen besteht.

7. Temporäre Abdeckschicht nach einem der vorstehenden Ansprüche, wobei der aktive Inhaltsstoff aus Kupfer, Silber und optional aus Gold besteht.

8. Temporäre Abdeckschicht nach einem der Ansprüche 1-7, wobei der aktive Inhaltsstoff Gold umfasst, vorzugsweise 1-10 Gewichtsprozent Gold.

9. Temporäre Abdeckschicht nach einem der vorstehenden Ansprüche, wobei der aktive Inhaltsstoff mindestens 15 Gewichtsprozent Kupfer und mindestens 15 Gewichtsprozent Silber umfasst.

10. Temporäre Abdeckschicht nach einem der vorstehenden Ansprüche, wobei der aktive Inhaltsstoff eine Mischung von Teilchen umfasst, umfassend die Vielzahl von Teilchen und Silberteilchen, wobei die Vielzahl von Teilchen optional Folgendes umfasst:
- 70-90 Gewichtsprozent Kupfer, vorzugsweise 75-85 Prozent, und
- 10-30 Gewichtsprozent Silber, vorzugsweise 15-25 Prozent.

11. Temporäre Abdeckschicht nach einem der vorstehenden Ansprüche, wobei der Körper aus flexiblem Material besteht.

12. Gemusterte Folie, umfassend eine oder mehrere temporäre Abdeckschichten nach einem der vorstehenden Ansprüche und optional eine oder mehrere Schwächungen zum Abnehmen der einen oder mehreren temporären Abdeckschichten.

13. Rolle von Klebeband, wobei das Klebeband die temporäre Abdeckschicht nach einem der Ansprüche 1-11 umfasst.

14. Verfahren zum Bereitstellen einer temporären Abdeckschicht zum Entfernen und/oder Inaktivieren von Mikroorganismen, wie Viren und/oder Bakterien, wobei das Verfahren Aufbringen einer Beschichtung, umfassend einen aktiven Inhaltsstoff, umfassend eine Vielzahl von Teilchen, aufweisend Kern, umfassend Kupfer, und eine Beschichtung, umfassend Silber, auf eine Stützschicht, umfassend eine Befestigungsoberfläche zum temporären Befestigen der Stützschicht an einer Oberfläche eines Objekts, umfasst.

15. Verwendung eines aktiven Inhaltsstoffs, umfassend eine Vielzahl von Teilchen, aufweisend einen Kern, umfassend Kupfer, und eine Beschichtung, umfassend Silber, in einer temporären Abdeckschicht zum Entfernen und/oder Inaktivieren von Mikroorganismen von einer Oberfläche eines Objekts, wobei die Verwendung optional eine Antifouling-Verwendung ist.

## Revendications

1. Couche de revêtement temporaire pour éliminer et/ou inactiver des micro-organismes, tels que des virus et/ou des bactéries, comprenant un corps présentant une surface de fixation pour fixer temporairement la couche de revêtement temporaire sur une surface d'un objet ; dans laquelle le corps présente une face comprenant un principe actif ; **caractérisée en ce que** le principe actif comprend une pluralité de particules présentant un noyau comprenant du cuivre et un revêtement comprenant de l'argent.

2. Couche de revêtement temporaire selon la revendication 1, dans laquelle la surface de fixation est une surface adhésive, facultativement formée d'un adhésif amovible pour détacher la couche de revêtement temporaire de l'objet.

3. Couche de revêtement temporaire selon l'une quelconque des revendications précédentes, dans laquelle le corps comprend une couche de support et un revêtement appliqué sur la couche de support ; dans laquelle la couche de support comprend la surface de fixation et le revêtement comprend le principe actif.

4. Couche de revêtement temporaire selon la revendication 3, dans laquelle le revêtement comprend un liant pour lier le principe actif à la couche de support, le liant étant facultativement sélectionné dans un groupe consistant en un alkyde, du polyméthacrylate de méthyle (PMMA) et du polyuréthane.

5. Couche de revêtement temporaire selon la revendication 3 ou la revendication 4, dans laquelle le revêtement présente une épaisseur de 100 micromètres ou moins ou de 10 micromètres ou plus.

6. Couche de revêtement temporaire selon l'une quelconque des revendications précédentes, dans laquelle la face consiste en ledit principe actif pour éliminer et/ou inactiver les micro-organismes.

7. Couche de revêtement temporaire selon l'une quelconque des revendications précédentes, dans laquelle le principe actif consiste en du cuivre, de l'argent et, facultativement, de l'or.

8. Couche de revêtement temporaire selon l'une quelconque des revendications 1 à 7, dans laquelle le principe actif comprend de l'or, de préférence 1 à 10 pour cent en poids d'or.

9. Couche de revêtement temporaire selon l'une quelconque des revendications précédentes, dans laquelle le principe actif comprend au moins 15 pour cent en poids de cuivre et au moins 15 pour cent en poids d'argent.

10. Couche de revêtement temporaire selon l'une quelconque des revendications précédentes, dans laquelle le principe actif comprend un mélange de particules comprenant ladite pluralité de particules et des particules d'argent, ladite pluralité de particules comprenant facultativement :
- 70 à 90 pour cent en poids de cuivre, de préférence 75 à 85 pour cent, et
- 10 à 30 pour cent en poids d'argent, de préférence 15 à 25 pour cent.

11. Couche de revêtement temporaire selon l'une quelconque des revendications précédentes, dans laquelle le corps est en matériau souple.

12. Feuille à motifs comprenant une ou plusieurs couches de revêtement temporaire selon l'une quelconque des revendications précédentes et, facultativement, une ou plusieurs fragilisations pour détacher la ou les couches de revêtement temporaire.

13. Rouleau de ruban adhésif, dans lequel le ruban adhésif comprend la couche de revêtement temporaire selon l'une quelconque des revendications 1 à 11.

14. Procédé destiné à fournir une couche de revêtement temporaire pour éliminer et/ou inactiver des micro-organismes, tels que des virus et/ou des bactéries, le procédé comprenant le fait d'appliquer un revêtement comprenant un principe actif comprenant une pluralité de particules présentant un noyau comprenant du cuivre et un revêtement comprenant de l'argent sur une couche de support comprenant une surface de fixation pour fixer temporairement la couche de support sur une surface d'un objet.

15. Utilisation d'un principe actif comprenant une pluralité de particules présentant un noyau comprenant du cuivre et un revêtement comprenant de l'argent dans une couche de revêtement temporaire pour éliminer et/ou inactiver des micro-organismes à partir d'une surface d'un objet, dans laquelle l'utilisation est facultativement une utilisation antisalissure.
